# EUROPEAN PATENT SPECIFICATION

(11) **EP 3 022 321 B1**
(45) Date of publication and mention of the grant of the patent: **13.01.2021**
(21) Application number: 14826682.8
(22) Date of filing: 16.07.2014
(51) Int. Cl.: C12Q 1/68, C07H 21/02

(54) **MIRROR BISULFITE ANALYSIS**
BISULFITSPIEGELANALYSE
ANALYSE MIROIR FAISANT APPEL AU BISULFITE

(30) Priority: 16.07.2013 US 201361847058 P
(43) Date of publication of application: 25.05.2016
(73) Proprietor: Zymo Research Corporation, Irvine, CA 92614 (US)
(72) Inventor: JIA, Xi-Yu, Newport Beach California 92660 (US); SUN, Xueguang, Tustin California 92614 (US)
(74) Representative: Hoppe, Georg Johannes
(86) International application number: PCT/US2014/046876
(87) International publication number: WO 2015/009844

(56) References cited:
- WO-A1-2011/127136
- WO-A1-2012/054730
- WO-A1-2012/119945
- WO-A1-2013/090588
- WO-A1-2013/090588
- US-A1- 2007 092 883
- US-A1- 2012 064 521
- MIGUEL R. BRANCO ET AL: "Uncovering the role of 5-hydroxymethylcytosine in the epigenome", NATURE REVIEWS GENETICS, 1 January 2011 (2011-01-01), XP055057585, ISSN: 1471-0056, DOI: 10.1038/nrg3080
- MULTIPLICOM.: 'Universal PCR for MiSeq Sequencing' 26 March 2013, XP008185902 Retrieved from the Internet: <URL:http://www.google.com/url?sa=t&rct=j&q =&esrc=s&source=web&cd=1&ved=0CB4QFjAA&url= ht tp%3A%2F%2Fwww.multiplicom.com%2Fsites%2Fde fault%2Ffiles%2FIFU018v1_IFU_PartII_M ID_MSq_0_0.pdf&ei=KVtiVJmcFa2ligLC8oHICw&us g=AFQjCNG93RoU5P7PJsEfLRI20MpJTp qnRw&sig2=XTqKpRoguMjCgv582qZOEw&bvm=bv.791 89006,d.cGE> [retrieved on 2014-11-10]
- ZYMO RESEARCH. 5-HMC GLUOSYLTRANSFERASE 27 May 2013, XP008182717 Retrieved from the Internet: <URL:https://web.archive.org/web/2013052709 3249/ http://www.zymoresearch.com/epigenetics/dna - hydroxymethylation/5-hmc-glucosyltransferas e/5-hmc-glucosyltransferase> [retrieved on 2013-11-10]

### SEQUENCE LISTING

<110> Zymo Research Corp.
<120> Mirror Bisulfite Analysis
<130> 1408
<150> 61847058
   <151> 2013-07-16
<160> 29
<170> PatentIn version 3.5
<210> 1
   <211> 60
   <212> DNA
   <213> synthetic
<400> 1
   aatgatacgg cgaccaccga gatctacact ctttccctac acgacgctct tccgatctcg 60
<210> 2
   <211> 65
   <212> DNA
   <213> synthetic
<400> 2
   gatcggaaga gcacacgtct gaactccagt caccgatgta tctcgtatgc cgtcttctgc 60
   ttggc 65
<210> 3
   <211> 34
   <212> DNA
   <213> synthetic
<220>
   <221> cytosines are methylated
   <222> (1)..(34)
<400> 3
   gtgactggag ttcagacgtg tgctcttccg atct 34
<210> 4
   <211> 47
   <212> DNA
   <213> synthetic
<400> 4
   aatgatacgg cgaccaccga gatctacact gacgacatgg ttctaca 47
<210> 5
   <211> 56
   <212> DNA
   <213> synthetic
<400> 5
   caagcagaag acggcatacg agatacgacg atactacggt agcagagact tggtct 56
<210> 6
   <211> 33
   <212> DNA
   <213> synthetic
<400> 6
   agatcggaag agcgtcgtgt agggaaagag tgt 33
<210> 7
   <211> 58
   <212> DNA
   <213> synthetic
<400> 7
   aatgatacgg cgaccaccga gatctacact ctttccctac acgacgctct tccgatct 58
<210> 8
   <211> 22
   <212> DNA
   <213> synthetic
<220>
   <221> cytosines are methylated
   <222> (1)..(22)
<400> 8
   tacggtagca gagacttggt ct 22
<210> 9
   <211> 64
   <212> DNA
   <213> synthetic
<400> 9
   gatcggaaga gcacacgtct gaactccagt caccgatgta tctcgtatgc cgtcttctgc 60
   ttgt 64
<210> 10
   <211> 69
   <212> DNA
   <213> synthetic
<400> 10
   acactgacga catggttcta caaatgatac ggcgaccacc gagatctaca ctgacgacat 60
   ggttctaca 69
<210> 11
   <211> 78
   <212> DNA
   <213> synthetic
<400> 11
   caagcagaag acggcatacg agatacgacg atactacggt agcagagact tggtcttacg 60
   gtagcagaga cttggtct 78
<210> 12
   <211> 104
   <212> **DNA**
   <213> **synthetic**
<400> 12
   gttacgcaaa ggaagaccaa gagtatggtg gtcactcgac ccaaaggaat atchmggtcm 60
   cmgggtacga caggagtcag atagaaccag acccagagga ggcg 104
<210> 13
   <211> 26
   <212> DNA
   <213> synthetic
<400> 13
   cgcctcctct gggtctggtt ctatct 26
<210> 14
   <211> 31
   <212> DNA
   <213> synthetic
<400> 14
   caaaaaaaga ccaaaaatat aataatcact c 31
<210> 15
   <211> 33
   <212> DNA
   <213> synthetic
<400> 15
   gttttttttg ggtttggttt tatttgattt ttg 33
<210> 16
   <211> 35
   <212> DNA
   <213> synthetic
<400> 16
   ctaaacaact aacccctaaa ccaaaaaaaa acaac 35
<210> 17
   <211> 31
   <212> DNA
   <213> synthetic
<400> 17
   gattggaaga gtgttgtgta gggaaagagt g 31
<210> 18
   <211> 23
   <212> DNA
   <213> Escherichia coli
<400> 18
   catcgttgac gaacacaccg gtc 23
<210> 19
   <211> 23
   <212> DNA
   <213> Escherichia coli
<400> 19
   catcgttgac gaacacaccg gtc 23
<210> 20
   <211> 23
   <212> DNA
   <213> Escherichia coli
<400> 20
   cttcggtatc agcagtaccg gtc 23
<210> 21
   <211> 24
   <212> DNA
   <213> Escherichia coli
<400> 21
   gattgctatt agtgcctccg gtgc 24
<210> 22
   <211> 24
   <212> DNA
   <213> Escherichia coli
<220>
   <221> hydroxymethylcytosine
   <222> (19)..(19)
<400> 22
   gattgctatt agtgcctccg gtgc 24
<210> 23
   <211> 21
   <212> DNA
   <213> Escherichia coli
<400> 23
   ctgtgatcca tcccggccat c 21
<210> 24
   <211> 23
   <212> DNA
   <213> Escherichia coli
<400> 24
   gaaacctgag ttgccggaac ttc 23
<210> 25
   <211> 23
   <212> DNA
   <213> Escherichia coli
<220>
   <221> hydroxymethylcytosine
   <222> (15)..(15)
<400> 25
   gaaacctgag ttgccggaac ttc 23
<210> 26
   <211> 22
   <212> DNA
   <213> Escherichia coli
<400> 26
   ccagcaaaac ccggacgtta tc 22
<210> 27
   <211> 21
   <212> DNA
   <213> Escherichia coli
<400> 27
   ggtgaaagtt ccggatggca c 21
<210> 28
   <211> 21
   <212> DNA
   <213> Escherichia coli
<220>
   <221> hydroxymethylcytosine
   <222> (12)..(12)
<400> 28
   ggtgaaagtt ccggatggca c 21
<210> 29
   <211> 21
   <212> DNA
   <213> Escherichia coli
<400> 29
   ctgagtttcg ccggtgagat g 21

## Description

This application claims the benefit of United States Provisional Patent Application No. 61/847,058, filed July 16, 2013.

### FIELD OF THE INVENTION

The present invention relates generally to the field of molecular biology, epigenetics and detection at the single nucleotide resolution for hydroxymethylcytosine residues. More particularly, it concerns methods for positive selection and quantitative determination of hydroxymethylcytosine frequency in the genome of humans and other species.

### DESCRIPTION OF RELATED ART

Epigenetic modifications are regarded as fundamental elements in gene expression regulation and include DNA methylation and hydroxymethylation. DNA methylation has been studied extensively and shown to play important roles in widespread biological phenomena, notable as a leading cause of human cancer (Jones and Laird, 1999). Though the existence of 5'-hydroxymethylcytosine (5hmC) was known to exist in T-even bacteriophages (i.e. T2/T4/T6) more recently, this modified base was reported anew and was identified as in mammalian tissue being found at higher levels in brain and neurons (Kornberg S. A. et al. 1961; Tahiliani, M. et al., 2009; Guo, J. G., et al. 2011; Kinney, S.M. et al, 2011).

As a result of the increased study of 5hmC, increasingly more sensitive tools are required for detection, quantitation, and ultimate mapping of the mark across the genome. However the precise role of 5hmC in the genomic context is under intensive study. One model implicates 5hmC in the oxidative demethylation of cytosine, which has been bolstered by subsequent characterizations of the 5-formylcytosine (5fC) and 5-carboxylcytosine (5caC) in the genome (Globisch D. et al., 2010; He, Y-F. et al., 2011). Aside from its mechanistic characterization, its localization and tissue distribution have also been closely studied, resulting in clear demonstration of elevated abundance in CNS tissues (Globisch D. et al., 2010). Pathologically, a profound depletion of 5hmC is observed across several malignant carcinomas (Jin S., et al., 2011)

As a result of the increased study of 5hmC, increasingly more sensitive tools are required for detection, quantitation, and ultimate mapping of the mark across the genome. U.S. Patent Application 12/963,272 filed 12/08/2010; entitled Detection of DNA Hydroxymethylation discloses methods for detecting and quantifying 5hmC Document WO2012/119945 discloses a method for detection of hydroxymethyl cytosine in a nucleic acid molecule preparation.

Document WO2013/090588 discloses a method and a kit for detection of 5-hydroxymethyl cytosine and /or 5-methylcytosine in a nucleic acid. U.S. Patent Application 13/392.286, entitled Detection and Quantification of Hydroxymethylated Nucleotides in a Polynucleotide Preparation discloses methods for detecting and quantifying 5hmC. However these disclosures do not teach methods for a positive identification system or selection of hydroxymethycytosine residues or efficient genomic library preparation for such analysis. U.S. Patent Application 13/933,051, entitled System for Positive Identification of Hydroxymethylation discloses a Reduced Representation Hydorxymethylation Profiling (RRHP) system that is only semi-quantitative and is somewhat enrichment based.

While methods such as LC-MS/MS-MRM are useful for sensitive detection and quantitation of this and other modified nucleosides, most genetic applications require the ability to pin down the mark to a tight region, locus, or specific junction within a locus. Several methodologies have become available that both revisit old methodologies, such as immunoprecipitation or qPCR, as well as some that take advantage of new methodologies including chemical labeling, single-molecule kinetic monitoring, nanopore conductivity, and more (Branco M.R., et al., 2012).

For many applications, genome-wide approaches including hMeDIP and bioorthogonal labeling with glucosylation provide robust enrichment pools for Sanger sequencing as well as massively parallel sequencing (i.e. Next-Gen Sequencing). Despite good coverage of the genome and high specificities, these methods are often profoundly limited by input requirements, typically in the neighborhoods of several micrograms. Such high amounts of DNA are often not feasible for investigations from precious samples like stem cells, or selectively isolated cellular subpopulations. Importantly, these enrichment-based methodologies are limited in their scope of resolution. For highly optimized protocols, identification of hydroxymethylated sites will fall within the range from several hundred bp to several kb. Depending on how well a particular region is annotated, such resolution is often insufficient to describe activity in transcriptionally relevant sites with confidence. The findings from such studies often require subsequent work with locus-specific assays such as glucMS-qPCR to validate specific 5hmC positions.

Previously, only global quantification of this base was possible, using such techniques such as HPLC, thin layer chromatography (TLC), and LC/MS. Site specific detection or sequence context detection of 5hmC has been a challenge because existing techniques to study 5'-methylcytosine (5mC) in a site specific manner (bisulfite conversion) cannot distinguish between 5mC and 5hmC. Further, enrichment methods utilizing J-base binding protein (JBP1) or antibodies or TET1 protein are unable to reach to single nucleotide resolution (Robertson et al, 2012).

Recently, sequence-level resolution of 5hmC positions have been reported though this is open to question for reliability (Booth, M. J. et al., 2011). The oxidative bisfulite sequencing (oxBS-Seq) takes advantage of selective chemical oxidation via organometallic catalysis to yield 5fC from 5hmC, which is then susceptible to traditional bisulfite conversion and results in a different signal from the 5mC, whereas the Tet-assisted bisulfite sequencing (TAB-Seq) employs Tet enzyme to oxidize 5mC into 5caC which is then subjected to bisulfite treatment. Both C and 5caU will be converted into uracil or its derivative, only 5hmC which is protected by glucose through the upfront β-GT reaction will survive to be detected. However, both methods have certain limitations although they could confer single base resolution. First both of them require as much DNA input as enrichment-based methods. Secondly their workflows are very complicated, involved multiple steps of chemical modification which not only destroy the majority of the original DNA, but also cause the false positive calling due to the uncompleted oxidation/conversion. At last both of them require very deep sequencing in order to detect 5hmC with low abundance.

Thus improved and true single base resolution quantitative and efficient methods to profile 5hmC for whole genomes as well as for loci targeted analysis are required.

### SUMMARY

The present invention is defined by the appended claims. In a first embodiment there is provided a method for identifying hydroxymethylcytosine (5hmC) residues in a DNA sample comprising synthesizing a DNA strand complementary to each parental DNA strand of a DNA sample; modifying one or more cytosine residue (C) in each of the parental DNA strands, wherein the cytosine residue adjacent to the modified cytosine residue and present on the opposite parental DNA strand remains unmodified; preferentially amplifying the newly synthesized strands from the DNA sample generating amplification products; analyzing each of the amplification products; and identifying the hydroxymethylcytosine residues in each of the amplification products. In certain aspects the synthesizing of the DNA strand complementary to each parental DNA strand of a DNA sample is performed with a primer with cytosine residues methylated. The methylated primer may be a P7 primer (SEQ ID NO: 2) (Illumina) or PE2 (SEQ ID NO: 8) (Fluidigm) other commercially available primer or custom designed primer containing a plurality of cytosine residues that are compatible with analysis including Next-Gen sequence analysis. In certain embodiments the modifying of one or more cytosine residues in each of the parental DNA strands comprises contacting the DNA sample with at least one modifying agent.

In some embodiments the modifying agents can be a glyclosyltransferase, a methyltransferase, and bisulfite. In certain aspects the glyclosyltransferase can be a glycosyltransferase-A (GT-A) family member and a glycosyltransferase-B (GT-B) family member. In other aspects the glyclosyltransferase may be a hexosyltransferase, a pentosyltransferase, and a sialyltransferase. In other aspects the hexosyltransferase can be a glucosyltransferase, a galactosyltransferase, a glucuronosyltransferase, a fucosyltransferase, a mannosyltrasnsferase, and a peptidoglycan glycosyltransferase. In a further aspect the pentosyltransferase can be an ADP-ribosyltransferase, a phosphoribosyltransferase, xylosyltransferase, and an arabinosyltransferase. In certain aspects the sialyltransferase can be a β-galactoside α-2, 6-sialyltransferase and a monosialoganglioside sialyltransferase. In still other aspects the glucosyltransferase can be a HMC α- glucosyltransferase or a HMC β-glucosyltransferase, can be a T6-glucosyl-HMC-β-glucosyltransferase. In a further aspect the glucosyltransferase can be isolated from a T2, T4, or T6 bacteriophage.

In many embodiments the DNA sample further comprises a plurality of genomic DNA fragments. In some embodiments the genomic DNA fragments each contain at least one adapter on each end. In preferred embodiments the adapters are commercially available and can consist of P5 (SEQ ID NO: 1), P7 (SEQ ID NO: 2), or a modified P5 (SEQ ID NO: 7) or modified P7 adapter (SEQ ID NO: 9) and other commercially available adapters or custom designed adapters.

In certain aspects the one or more cytosine residues in each of the parental DNA strands of a DNA sample is in the context of a CpG or GpC sequence. In other aspects the genomic DNA sample comprises fragments of a size selected from the group consisting of about about 1 bp to about 250 bp, about 50 bp to about 250 bp, about 50 bp to about 350 bp, about 100 bp to about 350 bp, about 250 bp to about 350 bp, about 100 bp to about 500 bp, and about 100 bp to about 700 bp. In preferred embodiments the genomic DNA sample comprises fragments of about 1 bp to BOUT 150 bp and about 150 bp to about 350 bp. In a further aspect the methyltransferase can consist of but is not limited to M.SssI and M.CviPI.

In specific embodiments the DNA sample is modified first with a glucosyltransferase, second with a methyltransferase and third with bisulfite. In specific embodiments preferentially amplifying the newly synthesized strands from the DNA sample generating amplification products further comprises preferentially amplifying the daughter strand with a forward and a reverse primer. In other embodiments the forward and reverse primers is a P5 primer (SEQ ID NO: 1) and a P7 primer (SEQ ID NO: 2), a CS1 primer (SEQ ID NO: 3) or a CS2 primer (SEQ ID NO: 4) (Fluidigm) and other commercially available sequencing primers forward and reverse primers. In some aspects the step of preferentially amplifying the newly synthesized strands from the DNA sample is performed with one or more sets of locus specific primers generating one or more locus specific amplicon (i.e. amplified with forward and reverse primers specific to a particular locus or loci). In certain aspects amplification with locus specific primers further comprises performing the synthesis with one or more methylated primers with a universal or optimized sequence of at least 20 bp to about 32 bp at the 5 prime end which is used for preferential amplification of the daughter strand.

In specific embodiments the step of identifying the hydroxymethylcytosine residues in each of the amplification products further comprises mapping sequence reads to a reference genome with a computer with software wherein sequence information from the newly synthesized strand identifies the hydroxymethylcytosine residues on the parent strand. In certain aspects the analysis of hydroxymethylcytosine residues with a computer further comprises quantitating the amount of hydroxymethylcytosine residues in the DNA sample. In still other aspects the analysis of hydroxymethylcytosine residues further comprises comparing a disease or variant sample and a normal sample, wherein differences between the disease or variant sample and the normal sample correlates with a phenotype, condition or disease. In still other aspects the analysis of hydroxymethylcytosine residues further comprises comparing a disease sample and a normal sample.

In embodiments identifying hydroxymethylcytosine residues in a DNA sample comprises merely modifying at least one cytosine residue in a DNA that further comprises two DNA strands; and determining if one or more of the at least one cytosine is hydrohymethylcytosine by examining the status of an adjacent cytosine on an opposite DNA strand in relation to the cytosine being examined.

In some embodiments the method for identifying hydroxymethycytosine residues in a DNA sample comprises synthesizing a DNA strand complementary to each parental DNA strand of a DNA sample; contacting the DNA sample with a glucosyltransferase, wherein hydroxymethylcytosine residues have at least one glucose added generating glucosyl-hydroxymethycytosine residues; contacting the DNA sample with at least one methyltransferase, wherein unmodified cytosine residues have a methyl group added, except for cytosine residues adjacent to glucosyl-hydroxymethylcytosine residues and on the opposite parental DNA strand which remain unmethylated; contacting the DNA sample with bisulfite, wherein unmodified cytosine residues are converted to uracil; preferentially amplifying the newly synthesized strands from the DNA sample with primers generating amplification products; sequencing each of the amplification products; identifying the hydroxymethylcytosine residues in each of the amplification products. In certain aspects the synthesis of the DNA strand complementary to each parental DNA strand is performed with a biotinylated primer. In other aspects embodiments for synthesizing each parental DNA strand with a biointylated primer further comprises pulling down the plurality of synthesized strands after the step of contacting the DNA sample with at least one methyltransferase.

DNA such as genomic DNA can be isolated from one or more cells, bodily fluids or tissues. An array of methods can be used to isolate DNA from samples such as blood, sweat, tears, lymph, urine, saliva, semen, cerebrospinal fluid, feces or amniotic fluid among others tissue or fluid sources. DNA can also be obtained from one or more cell or tissue in primary culture, in a propagated cell line, a fixed archival sample, forensic sample or archeological sample. Methods for isolating genomic DNA from a cell, fluid or tissue are well known in the art (See, e.g., Sambrook et al., 2001).

In preferred embodiments the plurality of genomic DNA fragments is mammalian genomic DNA. In other preferred embodiments the mammalian genomic DNA is human genomic DNA. In specific aspects the human genomic DNA is from a human subject or from a cell line or tissue bank or from a tissue biopsy, blood, urine, saliva or skin. In still other preferred embodiments the cultured cells are neuronal cells or stem cells. In specific preferred embodiments the human genomic DNA is from brain cells or tissue.

In certain aspects a kit of parts is provided for making a DNA library for identifying or quantitating hydroxymethylcytosine comprising: one or more enzymes (e.g. dsDNA Sherase Plus (Zymo Research Corp.) or restriction emnzymes such as BpaI, BfuC I, Csp6i, Hpa II, Msp I, MseI, Taq I, Csp6i, and or other Type II or Type III restriction enzymes, New England Biolabs-NEB) or chemical agents (Hydroxyl radicals) or physical means (shearing, French press or other means) for fragmenting genomic DNA to a desired size range (e.g. 100-500bp); a methylated primer, wherein the primer is at least partially complementary to a commercially available adapter (e.g. P7/P5, modified P7/P5 adapter or CS1/CS2, PE1/PE2, Fluidigm); a Glucosyltrnasferase enzyme; a Glucosyltrnasferase reaction buffer; a Uridine; Diphosphoglucose (UDGP) solution; a methylatransferase; a methylatransferase reaction buffer; at least one adapter pair (e.g. P5/P7; modified P7/P5 adapter CS1/CS2, PE1/PE2 or other); a ligase enzyme (e.g. T4 DNA ligase); optionally a ligase reaction buffer; at least one bisulfite compatable primer pair for PCR amplification and Next-Gen Sequencing (e.g. P5/P7; modified P5/P7, CS1/CS2, PE1/PE2); a thermopolymerase; optionally a thermopolymerase reaction buffer; a dNTP solution; a plurality of columns or plates comprising columns, a plurality of collections tubes or collection plates, a DNA Wash buffer, a ADP buffer, a DNA binding buffer; a DNA elution buffer; access to a suite of bioinformatics tools; and instructions for use. In certain aspects any component listed above for kits may be made optional in specific configurations.

As used herein, "a" or "an" may mean one or more. As used herein in the claim(s), when used in conjunction with the word "comprising", the words "a" or "an" may mean one or more than one.

The use of the term "or" in the claims means "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the description of the illustrative embodiments and the specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE DRAWINGS

**FIG. 1****:** Shown schematically is a representation of hydroxymethylcytosine analysis using single strand extension method (Mirror™ bisulfite sequencing).
**FIG. 2****:** Shown schematically is a representation of a workflow for making libraries for identifying hydroxymethylcytosine residues.
**FIG. 3****:** Shown is identification of hydroxymethylcytosine using synthetic oligonucleotides.
**FIG. 4****:** Shown is identification of hydroxymethylcytosine using locus specific analysis.
**FIG. 5****:** Shown is identification of hydroxymethylcytosine using locus specific analysis using a primer with a universal or optimized tail.
**FIG. 6****:** Shown is representative DNA samples made from single strand hydroxymethylation methods.
**FIG. 7****:** Shown are representative DNA libraries made with differing inputs of genomic DNA. (A) Gel image of completed libraries prepared from different inputs of 400 ng, 200 ng, 100 ng and 50 ng genomic DNA (left to right). (B) Library prepared from 50 ng input with an additional two cycles of amplification (bottom). "S" indicates short fragments that were gel selected from about 150bp to about 250bp and "L" indicates long fragments that were gel selected from about 250bp to about 350bp.

### DESCRIPTION OF ILLUSTRATIVE EMBODIMENTS

### I. Detection of Hydroxymethylation.

An illustrative and non-limiting protocol for hydroxymethylation analysis according to the invention is exemplified below.

Genomic DNA hydroxymethylation is of rapidly increased interest and focus within the Epigenetic field alongside methylation analysis (Tahiliani M. et al., 2009). However, to date there is no method to efficiently quantify with positive selection 5-hydroxymethylcytosine (5hmC) genome wide to the single nucleotide level. Novel methods are provided that both avoids complicated chemical conversion processes and affords sequence-level resolution of 5hmC positions for both genome wide analysis as well as locus specific analysis. It is also contemplated that other base modifications for adenosine and cytosine may be analyzed similarly to the methods disclosed for 5hmC. For example one skilled in the art could through routine changes to the methodology disclosed and with routine optimization well within the skill of one skilled in the art in light of the state of the art for N6-methyladenosine (6mA) (Xiao-Li Ping et al. 2014) and 5-formylcytosine (5fC) or 5-carboxylcytosine (5caC) (Ito S. et. al. 2011; Y.-F. He et al. 2011) analyze such added base modifications and which represent alternate embodiments.

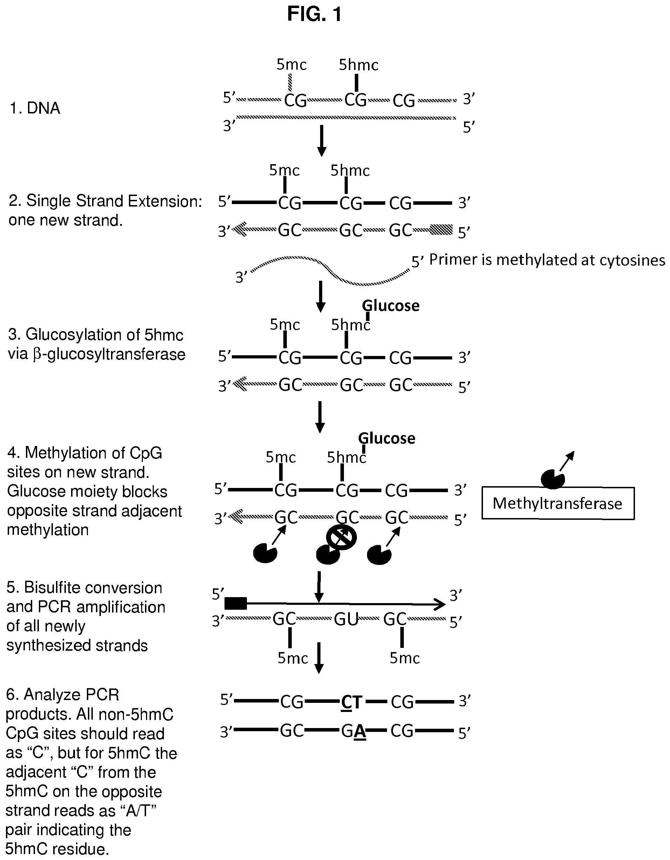

Referring to FIG. 1, schematically shown is a representative embodiment overview for a Mirror™ bisulfite analysis method for 5hmC identification and quantification set forth in Steps 1-6 of this illustrative embodiment. DNA from synthetic or biological sources is obtained (Step 1). In the case of genomic DNA it is typically obtained as fragments for example by any means known in the art such as digestion with restriction enzymes (e.g. dsDNA Sherase Plus, Zymo Research or restriction enzymes such as BpaI, BfuC I, Csp6i, Hpa II, Msp I, MseI, Taq I, Csp6i, and or other Type II or Type III restriction enzymes, New England BioLabs, Inc.) or chemical agents (hydroxyl radicals) or physical means (e.g. shearing, French press or other means) for fragmenting genomic DNA to a desired size range (e.g. 1to 150 bp. 150 to 350 bp or 100 to 500bp). Methods for chemical cleavage for massively parallel sequencing libraries are known in the art (Gyarmati, P. et al. 2013). Methods for physical cleavage for massively parallel sequencing libraries are also well known in the art.

In many embodiments the DNA sample further comprises a plurality of genomic DNA fragments. In some embodiments the genomic DNA fragments each contain at least one adapter on each end. In preferred embodiments the adapters are commercially available and can consist of P5 (SEQ ID NO: 1), P7 (SEQ ID NO: 2), or a modified P5 (SEQ ID NO: 7) or modified P7 adapter (SEQ ID NO: 9) and other commercially available adapters or custom designed adapters (not shown in FIG. 1).

In embodiments a primer is used to anneal and extend producing a single daughter DNA strand from the parental DNA strand, generally via the adapter (Step 2). In certain embodiments for making libraries (or locus specific analysis) the primer contains methylated cytosine residues and it at least partially compatible with adapters including but not limited to commercial adapters or modified adapters compatible with commercial adapters that have been added to the DNA fragments. In specific embodiments the primer contains at least 1, 2, 3, 4, 5, 6, 7, 8, 9, 10 or more methylated cytosine residues. In some embodiments it is preferred that at least one adapter contain few cytosine residues (e.g. 1, 2, 3, or 4 cytosine). In other embodiments the primer is a P7 primer (Illumina) (SEQ ID NO 2) or derivative primary sequence modified P7 primer (SEQ ID NO: 3) (i.e. P7 or modified P7 both with methylated cytosine residues, (SEQ ID NO: 3) or other commercially available primer with cytosine residues methylated. (e.g. Fluidigm CS1/CS2/PE1/PE2 primers/adapters (SEQ ID NO: 4)/ (SEQ ID NO: 5), sequence modifications of CS1/CS2 (SEQ ID NO: 10/ SEQ ID NO: 11) primers or other designed primers for commercial adapters). In some embodiments the primers also contains a universal or optimized tail of from about 10 to about 20 nucleotides or about 20 to about 30 nucleotides or about 30 to about 40 or more nucleotides for aiding in distinguishing the newly synthesized strand from the parental strand during the post bisulfite treatment amplification step. In one embodiment the universal or optimized tail about 24 to about 28 nucleotides or 22 to about 32 nucleotides.

Exemplary DNA samples that can be used in a method of the invention include, without limitation, mammal DNA such as a rodent, mouse, rat, rabbit, guinea pig, ungulate, horse, sheep, pig, goat, cow, cat, dog, primate, human or non-human primate. Plant DNA may also be analyzed according to the invention. For example, DNA from Arabidopsis thaliana, maize, sorghum, oat, wheat, rice, canola, or soybean may be analyzed. It is further contemplated that genomic DNA from other organisms such as algae, a nematodes, insects (e.g., Drosophila melanogaster, mosquito, fruit fly, honey bee or spider), fish, reptiles, amphibians and yeast may be analyzed.

In certain embodiments, DNA samples for use according to the invention comprise at least a first 5hmC position that has been modified by the addition of a bulky chemical moiety by an agent. Examples of bulky chemical moieties include, but are not limited to, hydrocarbon chains, aromatic rings, saturated and unsaturated lipids, sugars, polysaccharides and amino acids. For instance, 5'hmC positions may be glycosylated, such as by additional of a glucose moiety (i.e., glucosylated). Thus, according to certain aspects of the invention, 5hmC positions in sample DNA are modified by a chemical or enzymatic process. In some aspects, a DNA sample is treated with an enzyme to glycosylate 5hmC.

In certain embodiments the DNA sample is modified with an agent, for example glycosylated by treating with an enzyme such as but not limited to a glycosyltransferase (e.g. glycosyltransferase: GT-A or GT-B family member) or preferentially a glucosyltransferase.

In this embodiment DNA is treated with a glucosyltransferase, e.g. β-gucotransferase or α-glucosyltransferase to add a glucose residue (Kornberg S.R. et al., 1961) (Step 3). In another embodiment a diglucosyl moiety can be added by converting a mono-glucosyl residue to a diglucosyl residue (e.g. β-Glucosly-α-glucosyl) by treating first with β-glucosyl transferase followed by treating with Glucosyl-β-glucosyl transferase (i.e. T6-glucosyl-HMC-b-glucosyl transferase). In other embodiments the substrate for the glucosyl transferase (UDPG: uracil-diphosphate glucose, or UDP-glucose) can be made into a derivative changing or modifying the sugar added (e.g. galactose, fucose, mannose, maltose, and like sugars that are expected to inhibit methyltransferase or restriction endonuclease cleavage similarly).

Thibodeaux, C. et al. discloses in the journal Nature a review entitled "Unusual sugar biosynthesis and natural product glycodiversification" (incorporated in its entirety by this reference), that the sequences of more than 100 gene clusters involved in production of glycosylated natural products are known as are the activities of numerous sugar biosynthetic enzymes together with well characterized glycosyltransferases. It is thus within the skill of the art to use such gene/enzymes systems to engineer glycosyl transferases from their natural specificities to alternate sugar specificities that only depends on the tolerance of particular glycosyltransferases for alternate sugar substrates (Thibodeaux, C. et al.). Alternate embodiments thus contemplate using glycosyltransferase or engineered glycosyltransferase to generate hydroxymethylcytosine residues with alternate sugar moieties (e.g. galactose, fucose, mannose, maltose and like sugars).

A variety of methods can be used to analyze DNA molecules comprising a protected 5'Glu-hmC position. For example, glucosylated DNA can be hybridized to an array to determine the sequences of hydroxymethylated positions in DNA samples. DNA molecules comprising a 5'Glu-hmC were also found to serve a suitable template for DNA polymerase (See e.g., Example 6). Accordingly, protected DNA molecules can also by analyzed by PCR-based techniques or by direct DNA sequencing. Furthermore, modified 5'Glu-hmC may provide antibody-binding target and 5'Glu-hmC-binding antibodies may exhibit enhanced specificity and binding affinity relative to antibodies that bind to 5'hmC. Accordingly, 5'Glu-hmC-binding antibodies can be used in improved methods DNA hydroxymethylation analysis.

In embodiment the DNA sample is treated with a methyltransferase. In some embodiments the methyltransferate may be CpG Methyltransferase (M.SssI), GpC Methyltransferase (M.CviPI), Dnmt1, Dnmt2, Dnmt3a, Dnmt3b, HaeIII, Methyltransferase, AluI Methyltransferase, HpaII Methyltransferase, MspI Methyltransferase, BamHI Methyltransferase, HhaI Methyltransferase, BsuRIa Methyltransferase, BsuRIb Methyltransferase, BssHII Methyltransferase, MarII Methyltransferase, SinI DNA Methyltransferase, AcII Methyltransferase, and BssHII Methyltranferase. In specific embodiments the methyltransferase can be of M.SssI and M.CviPI and like enzymes. In certain aspects the modification of the 5hmC by addition of glucose to form 5hmC (or other moiety) or other modification will inhibit methylation of an adjacent cytosine residue on the opposite DNA strand (e.g. Cytosine residues in a CpG or GpC context) (Step 4). Hydroxymethylation typically occurs in gene bodies as well as in CpG containing nucleic acid. The CpG containing nucleic acid may be present in, *e.g*., in a CpG island, a CpG doublet, a promoter, an intron, or an exon. For instance, in the genetic regions provided herein the potential hydroxymethylation sites encompass the promoter/enhancer regions of the indicated genes. Thus, the regions can begin upstream of a gene promoter and extend downstream into the transcribed region. In certain specific aspects DNA methylation is determined one or more of the genes or genomic regions. For example, for the genomic regions provided in representative genomic DNA, hydroxymethylation can be determined at each CpG and a quantitative number calculated for each region.

In embodiments, the DNA sample is treated with bisulfite according to standard commercially available protocols thereby converting all unmodified cytosine residues (C's) to uracil (U) which read as thymine residues (T's) in subsequent analysis while methylated cytosine residues (5mC), hydroxymethylcytosine (5hmC) or glucosylated hydroxymethylcytosine (5hmC^{-Glucose}) remain unchanged (Step 5). In some embodiments the modified DNA for example glucosylated DNA is purified before subsequent steps of treating with bisulfite (not shown in FIG. 1). In other embodiments proteinases (e.g. Proteinase K), detergents (e.g. Triton-x-100), solvents (e.g. Polar protic solvents: methanol, ethanol or Polar aprotic solvents: acetone dimethylformamide) chaotropes (e.g. Guanidine thiocyanate, GTC) may be used to treat the modified DNA before purification before purification to aid in recovery before bisulfite treatment.

Many methods for detecting the presence, absence, or amount of methylation or hydroxymethylation involve exposing genomic DNA to bisulfite (Step 5). Exposure of DNA, such as genomic DNA, to bisulfite does not affect methylated cytosine in a CpG, while unmethylated cytosines are changed to uracil. Thus, many methods for detecting methylation or hydroxymethylation of a gene involve the treatment of DNA with bisulfite, and subsequent analysis of the resulting nucleotide. Techniques for bisulfate conversion of DNA are well known in the art and commercial kits that provide optimized conversion efficiency are available (e.g., the EZ DNA Methylation-Lightning® or -Gold, -Direct, -Start-Up, kits available from Zymo Research Corp., Irvine, CA). Various methods for detecting methylation are disclosed, e.g., in PCT Pub. No. WO/2010/114821; PCT Pub. No. WO/2011/109S29; U.S. 2011/0046009, U.S. 2010/0304992, U.S. Pat. No. 5,786,146; Fraga, 2002; El-Maarri, 2003; Laird, 2003; and Callinan, 2006.

In most embodiments the bisulfite converted DNA sample fragments are analyzed for example by Next-Gen sequencing (Illumina) or other commercially available platform capable of analyzing DNA to determine its sequence.(e.g. Life Technologies Ion Torrent™ or Ion Proton™; Roche 454 pyrosequencing, Nanopore, microarray, bead array, other hybridization based microarray, or probe based detection). All non-5hmC CpG sites should read as "C", but for 5hmC on the adjacent "C" from the 5hmC on the opposite strand reads as "A/T" pair indicating the 5hmC residue. In some embodiments computer aided software facilitated mapping and identification as well as quantitation of 5hmC. In still other embodiments filtering is used with no β-GT controls to remove spurious false reads of A/T not resulting from a 5hmC residue in the parental DNA strand. In one aspect, methods of the embodiments concern determining the presence of hydroxymethylcytosine in a genomic sample to quantitate the distribution via use of analysis software and computers.

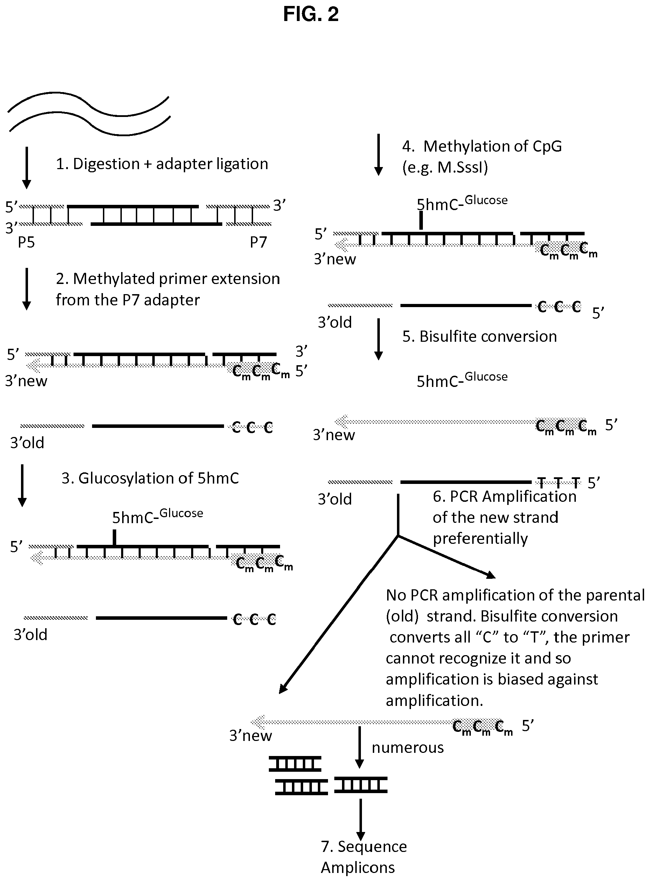

Referring now to FIG. 2, schematically shown is an embodiment for making genomic DNA libraries for genome wide and whole genome analysis in Steps 1-7 (See Example 2). In step 1 genomic DNA is digested with one or more enzymes or uniformly fragmented by other means generating a plurality of DNA fragments (i.e. by enzymatic, chemical means orphysical). Adapters including to but not limited to commercially available adapters for Next Generation (Next-Gen) sequencing (e.g. P5/P7, TruSeq, Illumina (SEQ IO NO: 1/SEQ ID NO 2); modified P5/P7 (SEQ IO NO: 9/SEQ ID NO 10); CS1/CS2 (SEQ IO NO: 3/SEQ ID NO 4); modified CS1/CS2 (SEQ IO NO: 12/SEQ ID NO:13) Access Array for amplicon tagging, Fluidigm Corp. or other adapter tag) are added for example by ligating to the DNA fragment ends, added by PCR or by other means. In some embodiments it is preferred that at least one adapter contain few cytosine residues (e.g. only 1, 2, 3, or 4 cytosine). In this embodiment in step 2, a methylated primer (e.g. with a P7 primer with all Cytosine (C) residues methylated to 5mC) (SEQ ID NO: 3) is used to synthesize a single daughter strand for each DNA fragment. In step 3, the DNA sample is then treated to modify the DNA sample for example by treating with a glucosyltransferase (glucosylation) or other modifying enzymes where all 5hmC residues are modified to 5hmC-glucose or other moiety if using a different modifying agent (e.g. other enzyme). In step 4, the modified DNA sample is treated with at least one methyltransferase, for example by treating with M.sssI and M.CviPI and like enzymes. In other embodiments the DNA sample can be treated with one or more or different methyltransferases. In all such embodiments the methyltransferate may be selected from CpG Methyltransferase (M.SssI), GpC Methyltransferase (M.CviPI), Dnmt1, Dnmt2, Dnmt3a, Dnmt3b, HaeIII, Methyltransferase, AluI Methyltransferase, HpaII Methyltransferase, MspI Methyltransferase, BamHI Methyltransferase, HhaI Methyltransferase, BsuRIa Methyltransferase, BsuRIb Methyltransferase, BssHII Methyltransferase, MarII Methyltransferase, SinI DNA Methyltransferase, AcII Methyltransferase, and BssHII Methyltranferase as well as other known methyltransferases. As described above methylation of cytosine residues (C) is inhibited for adjacent C's on the opposite DNA strand in CpG or GpC contexts. In step 5, the DNA sample is treated with bisulfite converting unmodified C's to uracil (U) as described above. This bisulfite treatment converts C's in the parental DNA strand adapter regions rendering them unsuitable to amplification with primers. In step 6 the primers that are used to amplify preferentially the new daughter DNA strand are standard primers compatible with the adapter system (e.g. P5/P7 primers, Forward Primer, FP, and Reverse Primer, RP or other primers such as CS1/CS2, Fluidigm or custom designed primers compatible with commercial or custom adapters). In this embodiment there is little to no amplification of parental DNA strands due to incompatibility with converted sequence with the primers where all C's are converted to U which acts as a T residue for subsequent analysis and there is a profound bias for amplification of all newly modified daughter DNA strands producing a DNA library. In this step a minimum number of rounds of amplification is used in different embodiments and can be 1, 2, 3, 4, 5, 6, 7, 8, 9, 10, 11, 12, 13, 14, and 15 or more amplification rounds. In step 7 the DNA library is sequenced by commercially available means such as with massively parallel next generations sequencing for example on a Mi-Seq, Hi-Seq, (1500/2000/2500-Illumina) or analyzed on equivalent platforms (e.g. Life Technologies Ion Torrent™ or Ion Proton™; Roche 454 pyrosequencing, Nanopore, microarray, bead array, other hybridization based microarray, or probe based detection) (See Examples 2 and 3).

Different embodiments are compatible with other commercially available platform for example Ion Torrent™ Personal Genome Machine or Ion Proton™ (Life Technologies) semiconductor sequencing may be used to analyze genomic libraries or for locus specific 5hmC analysis. Roche 454 massively parallel pyrosequencing adapters may also be used similarly. In still different embodiments Nanopore, microarray, bead array, other hybridization based microarray, or probe based detection may be used. It is within the skill of the art to modify the above library methodology to any such commercially available or otherwise well-known platform, adapters or primers.

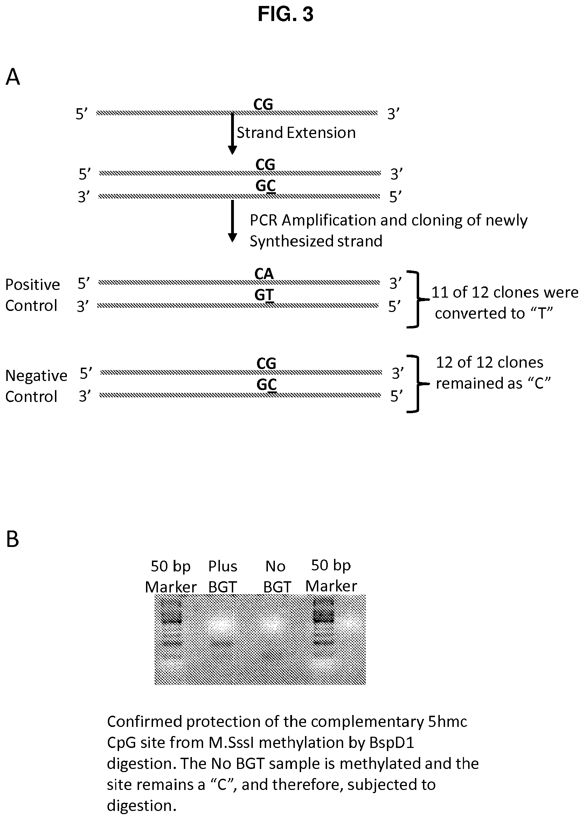

Referring now to FIG. 3, schematically shown are hypothetical synthetic oligo-nucleotides containing a 5hmc in a CpG context were used to validate the above methodology (See Example 1). In this assay samples are treated as described above with or without glucosyltransferase (β-GT) and cloned after the PCR amplification step. In FIG.3 11 of 12 amplicon clones showed for the positive control with glucosyltransferase (plus β-GT) conversion to T on the adjacent opposite strand in the CpG contect while 12 of 12 amplicon clones.

Referring now to FIG. 3, the sequencing of amplicons was confirmed with digestion where protection was seen from M.SssI methylation for adjacent complementary 5hmC CpG site. The negative control samples were methylated and the site remained a C and therefore was subject to digestion (See Example 1).

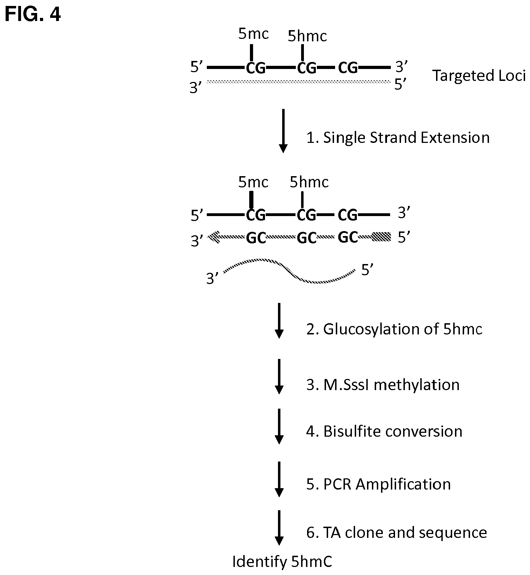

Referring now to FIG. 4, schematically shown is an embodiment for using the single strand 5hmC methodology for locus-specific analysis. In this embodiment any suitable genomic loci and a suitable primer can be chosen to perform the single daughter strand synthesis. In some embodiments the primer may be methylated. As described above, individual amplicon clones can be sequenced to identify 5hmC from information interpreted from adjacent non-converted C's on the opposite (complementary) strand efficiently identifying and quantitating all such 5hmc residues (See Example 4).

In an embodiment of the invention, the method of detecting a 5hmC in a DNA sample further includes recording in a computer-readable form detection data indicative of the occurrence of a 5hmC in the DNA sample. The detection data may be binary in the form of presence or absence of the hydroxymethylation site. The detection data may be a product of the calculations provided in the examples (See for example, Examples 5). The detection data may include an address corresponding to the characteristics of the polynucleotide fragment such as size, source, environmental context, age or developmental condition of the source as well as an identified genomic locus if available corresponding to the detection data for the polynucleotide preparation. The address may alternatively or additionally contain a code to identify the order in which data is inserted into a database and any weighting deemed applicable.

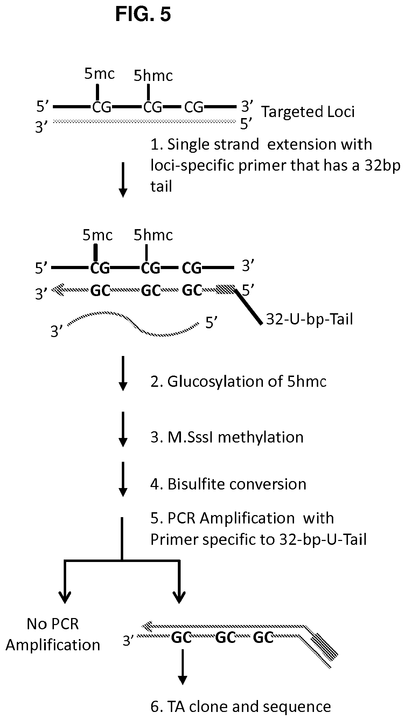

Referring now to FIG. 5, schematically shown are alternate embodiments using the single strand 5hmC methodology for locus-specific analysis using an about 32 bp primer tail. In such embodiments the single strand extension is performed with a loci-specific primer. In some embodiments this may be a universal or optimized random sequence from about 32 bp and from about 20 to about 32 bp, or about 25 bp to about 36 bp or longer sequence designed to reduce background amplification from spurious genomic loci. In certain embodiments this universal or optimized random sequence is used for preferential amplification of daughter strands in locus specific analysis. In such embodiments amplicon clones can be analyzed for example by sequencing and information interpreted from adjacent non-converted C's on the opposite (complementary) strand efficiently identifying and quantitating all such 5hmc residues.

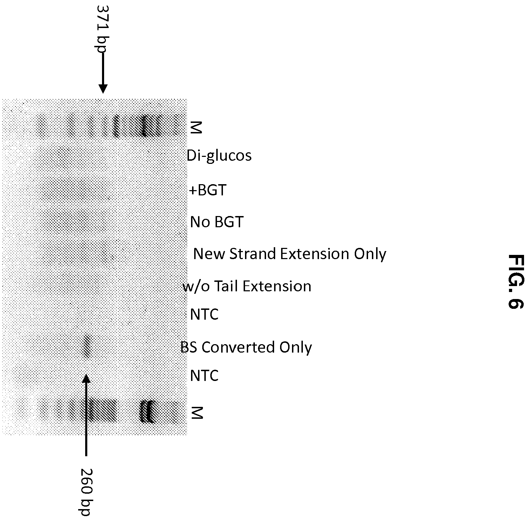

Referring now to FIG. 6, shown are representative DNA fragments made by the Mirror™ bisulfite analysis methods. Shown (left to right) are marker; genomic DNA fragments with a Di-glucose moiety added; treated with β-GT and with no treatment; newly synthesized daughter single strand DNA; bisulfite converted DNA only; NTC (non-transformed control); marker.

In other contemplated embodiments the single strand primer for synthesis or extension of the daughter strand can be methylated for its cytosine residues or can be labeled for example with biotin (biotinylated) or equivalently modified to provide preferential amplification of daughter strands or in some cases allowing pull down of the daughter strand for subsequent analysis. In this embodiment other steps remain essentially the same. In still other embodiments a uracil containing adapter may be used in conjunction with a suitable available glycosylase (e.g. Uracil-DNA glycosylase; UNG/UDG) to digest off the original parental adapter in the template for library clones which would not be able to be amplified (Longo, M. C. et al. 1990). In other embodiments adapters with recognition sequences for 5-Methylcytosine DNA glycosylase (Demeter recognition site: CG, CNG and CNN) may be similarly used which is disclosed in U.S. Patent Publications US 2009/0305241 A1, entitled "DNA Demethylases and Uses Thereof' published Dec 10, 2009 and US 2012/0149023 A1, entitled Engineered Demeter 5-Methylcytosine DNA Glycosylase with Improved Yield, Stability and Solubility, published June 14, 2012.

In certain embodiments, methods for determining the presence of hydroxymethylation involve adding oligonucleotide tags to adapter-fragments to generate tagged DNA(s). In certain aspects, oligonucleotide tag sequences comprise double stranded DNA having a known sequence, such a sequence that hybridizes to primers that can be used for DNA sequencing and/or PCR amplification. For example, methods for DNA methylation analysis by tagging cleaved DNA are known in the art and may be applied to methods according to the invention (see, e.g., WO/2010/114821). In specific embodiments the oligonucleotide tags are commercially available adapters (e.g. P5/P7 Illumina TruSeq or CS1/CS2 Fluidigm or modified derivatives thereof or other available or custom designed adapters or tags). In some aspects, oligonucleotide tag sequences comprise a label, such as a fluorescent label, a colorimetric label, a radioactive label, an antigen label, a sequence label, an enzymatic label or an affinity label (e.g., biotin). Thus, in certain cases, tagged DNA can be purified using the label, such as by using an avidin-biotin affinity column or affinity beads. A variety of commercially available ligase enzymes may be employed for ligating cleaved DNA to tags, including but not limited to, a bacterial DNA ligase or a phage DNA ligase (e.g., T4 DNA ligase). In further aspects, methods according to the invention further comprise treating the ligated (tagged) DNA with an enzyme that polymerizes additional 3' sequence, thereby repairing the 3' end of the tagged DNA. For example, a DNA polymerase such as Taq polymerase can be employed.

While the methodologies detailed above involve direct assessment of DNA hydroxymethylation status, it will be recognized that hydroxymethylation can also be indirectly assessed. For example, indirect assessment can comprise correlating hydroxymethylation and gene expression levels, thus hydroxymethylation status can be determined by determining the expression level of the indicated gene. Such information can be compared to quantitative hydroxymethylation status to confirm data from whole genome, genome wide or locus specific targeted analysis.

### Kits

The technology herein includes kits for evaluating presence, absence, or amount of hydroxymethylcytosine in one or more genes, gene regions or genomic regions. A "kit" refers to a combination of physical elements. For example, a kit may include, for example, one or more components such as probes, including without limitation specific primers, enzymes, reaction buffers, an instruction sheet, and other elements useful to practice the technology described herein. The kits may include one or more primers, such as primers for PCR, to detect methylation of one or more of the genes as described herein. These physical elements can be arranged in any way suitable for carrying out the invention.

Kits for analyzing hydroxymethylation of one or more genes may include, for example, a set of oligonucleotide probes for detecting hydroxymethylation in one or more gene or genomic regions. The probes can be provided on a solid support, as in an array (e.g., a microarray), or in separate containers. The kits can include a set of oligonucleotide primers useful for amplifying a set of genes described herein, such as to perform PCR analysis. Kits can include further buffers, enzymes, labeling compounds, and the like. Any of the compositions described herein may be comprised in a kit. The kit may further include water and hybridization buffer to facilitate hybridization of the two nucleic acid strands.

The components of the kits may be packaged either in aqueous media or in lyophilized form. The container means of the kits will generally include at least one vial, test tube, flask, bottle, syringe or other container means, into which a component may be placed, and preferably, suitably aliquoted. Where there is more than one component in the kit, the kit also will generally contain a second, third or other additional container into which the additional components may be separately placed. However, various combinations of components may be comprised in a single vial. The kits also will typically include a means for containing the nucleic acids, and any other reagent containers in close confinement for commercial sale. Such containers may include injection or blow molded plastic containers into which the desired vials are retained.

A kit will also include instructions for employing the kit components as well the use of any other reagent not included in the kit. Instructions may include variations that can be implemented. It is contemplated that such reagents are embodiments of kits of the invention. Such kits, however, are not limited to the particular items identified above and may include any reagent used for the manipulation or characterization of the methylation of a gene.

The container means of the kits will generally include at least one vial, test tube, flask, bottle, or other container means, into which a component may be placed, and preferably, suitably aliquoted. Where there is more than one component in the kit, the kit also will generally contain additional containers into which the additional components may be separately placed. However, various combinations of components may be comprised in a container. The kits of the present invention also will typically include a means for packaging the component containers in close confinement for commercial sale. Such packaging may include injection or blow-molded plastic containers into which the desired component containers are retained.

### Definitions

As used herein the term "gene or genes" refers to a region of genomic DNA associates with a given gene or genes. For example, the region can be defined by a particular gene (such as protein coding sequence exons, intervening introns and associated expression control sequences) and its flanking sequence. It is, however, recognized in the art that methylation in a particular region (e.g., at a given CpG position or in a amplification interval) is generally indicative of the methylation status at proximal genomic sites. Accordingly, determining a methylation status a gene region can comprise determining a methylation status at a site or sites within or flanking about 10 to about 50 bp, about 50 bp to about 100 bp, About 100 bp to about 200 bp, about 200 bp to about 300 bp, about 300 bp to about 400 bp, about 400 bp to about 500 bp, about 500 bp to about 600 bp, about 600 bp to about 700 bp, about 700 bp to about 800 bp, about 800 bp to about 900 bp, about 900 bp to about 1kb, about 1 kb to about 2 kb, about 2kb to about 5 kb, about 5 kb to about 10 kb, about 10 kb to about 20 kb, about 20 kb to about 50 kb or more of a named gene or CpG position.

As used herein the term "genomic region" refers to a region of genomic DNA not associated or not yet associated with a given gene. For example, the region can be defined by a particular genomic interval or a non-annotated region (such as protein coding sequence exons, intervening introns and associated expression control sequences or have other undetermined roles) and its flanking sequence. It is, however, recognized in the art that methylation in a particular genomic region (e.g., at a given CpG position or in an amplification interval) is generally indicative of the methylation status at proximal genomic sites. Accordingly, determining a methylation status a gene region can comprise determining a methylation status at a site or sites within or flanking about 10 to about 50 bp, about 50 bp to about 100 bp, about 100 bp to about 200 bp, about 200 bp to about 300 bp, about 300 bp to about 400 bp, about 400 bp to about 500 bp, about 500 bp to about 600 bp, about 600 bp to about 700 bp, bout 700 bp to about 800 bp, about 800 bp to about 900 bp, about 900 bp to about 1kb, about 1 kb to about 2 kb, about 2kb to about 5 kb, about 5 kb to about 10 kb, about 10 kb to about 20 kb, about 20 kb to about 50 kb or more of a CpG position in a genomic region.

As used herein "determining a hydroxymethylation status" for an indicated gene or gene region means determining whether one of more position in the DNA of the gene is hydroxymethylated. Thus, in certain aspects, determining a hydroxymethylation status for a gene comprises determining the methylation status at least 1, 2, 3, 4, 5, 6, 7, 8, 9, 10, 15, 20, 25, 30, 35, 40, 45, 50 or more sites of potential DNA hydroxymethylation. In certain aspects, a hydroxymethylation status is determined for a gene body or a promoter/enhancer region of a gene, of CpG island.

It is specifically contemplated that an individual component or element of a list may be specifically included or excluded from the claimed invention.

The use of the word "a" or "an" when used in conjunction with the term "comprising" in the claims and/or the specification may mean "one," but it is also consistent with the meaning of "one or more," "at least one," and "one or more than one."

Throughout this application, the term "about" is used to indicate that a value includes the standard deviation of error for the device or method being employed to determine the value.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description. Other embodiments of the invention are discussed throughout this application. Any embodiment discussed with respect to one aspect of the invention applies to other aspects of the invention as well and vice versa. The embodiments in the Example section are understood to be embodiments of the invention that are applicable to all aspects of the invention.

### VI. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention. Unless noted otherwise reference to commercial kits or protocols is performed according to the respective manufacturer's recommended protocol.

### Example 1 - Resolution of Single-Base 5hmC using Synthetic Oligo Nucleotides.

Purpose: Determine if glucosylation of 5hmC can block M.SssI methylation of complementary CpG site.
1. Primer extension of synthetic oligo: Sequence of 5hmC Oligo: (See: 5hmC; 5mC). SEQ ID NO: 13, Primer RK20: 5'CGCCTCCTCTGGGTCTGGTTCTATCT-3'.

| | | | |
|---|---|---|---|
| **C** | 4 µl | PCR Reactions | 1.) 95°C for 10:00 |
| **5hmC Oligo (20 µM)** | 4 µl | | 2.) 37°C for 15:00 |
| **ZymoTaq 2X MM** | 25 µl | | 3.) 72°C for 45:00 |
| **H₂O** | 17 µl | | |
| Total | 50 µl Rxn | | |

**2.** Clean-up with Oligo Clean & Concentrator. Elute in 15 µl H₂O (Zymo Research Corp.).
**3.** Glucosylation of 5hmC sites: Incubate O/N at 37°C. Clean-up with Oligo Clean & Concentrator. Elute in 15 µl (Zymo Research Corp.).

| | **Di-glucosylation** | **+BGT** | **Negative** |
|---|---|---|---|
| **10X GT Rxn Buffer** | 5 µl | 5 µl | 5 µl |
| **10X UDPG** | 5 µl | 5 µl | 5 µl |
| **βGT enzyme (2U/µl)** | 5 µl | 5 µl | - |
| **T6-βGT Enzyme** | 10 µl | - | - |
| **DNA (500 ng total)** | 1.25 µl | 1.25 µl | 1.25 µl |
| **H₂O** | 23.75 µl | 33.75 µl | 38.75 µl |
| **Total** | 50 µl | 50 µl | 50 µl |

**4.** Methylation of CpG sites: Incubate at 30°C for 4hrs and heat-inactivate at 65°C for 15mins.

| | |
|---|---|
| **10X CpG Rxn Buffer** | 2 µl |
| **20X SAM** | 1 µl |
| **DNA** | 13.5 µl |
| **CpG Methylase** | 1 µl |
| **H₂O** | 2.5 µl |
| Total | 20 µl Rxn |

**5.** Bisulfite Conversion: Add 130 µl bisulfite conversion reagent directly to the methylation reaction according to the manufacturer's recommended protocol for the EZ DNA Methylation Lightning® Kit (Zymo Research Corp.).
**6.** PCR amplification of bisulfite-converted, newly synthesized strand:
SEQ ID NO: 14, DT10 BS Fwd Primer:
5'CAAAAAAAGACCAAAAATATAATAATCACTC-3'
SEQ ID NO: 15, DT11 BS Rev Primer:
5' GTTTTTTTTGGGTTTGGTTTTATTTGATTTTTG-3'

| | | | | |
|---|---|---|---|---|
| **DNA** | 3 µl | PCR Reactions | 1.) 95°C for 10:00 | |
| **DT10 BS Fwd Primer (10 µM)** | 1 µl | | 2.) 95°C for 0:30 | \| |
| **DT11 BS Rev Primer (10 µM)** | 1 µl | | 3.) 50°C for 0:30 | \| X25 cycles |
| **ZymoTaq 2X MM** | 25 µl | | 4.) 72°C for 0:30 | \| |
| **H₂O** | 20 µl | | 5.) 72°C for 1:00 | |
| Total | 50 µl Rxn | | 6.) 4°C forever | |

**7.** Purify PCR products by DCC and elute in 15 µl elution buffer (Zymo Research Corp.).
**8.** TA clone remaining PCR product into pGEM T-easy vector and sequence colonies (Promega Corp.).

Glucosylation of 5hmC protects against methylation on the complementary synthesized strand, so the complementary cytosine is converted to a "T". Glucosylation (Di) had 9/10 conversion and β-GT had 11/12 conversion. The negative control was methylated and remained a "C". Therefore, we can conclude that the CpG of the parental strand is hydroxymethylated. (See FIG. 3).

### Example 2: 5hmC Library Preparation Protocol 1

Purpose: Prepare genomic libraries using the strand extension method coupled with enzyme-based modifications for high-throughput parallel sequencing. This method was used to detect 5hmC of human brain DNA (Zymo Research Corp. D5018).
1. Digestion and adapter ligation: Incubate at 21°C for 6hrs and 4°C overnight.

| | |
|---|---|
| **10X NEB Buffer 4** | 5 µl |
| **Mspl (20U/µl)** | 2 µl |
| **T4 Ligase (2000U/µl)** | 1 µl |
| **ATP (10mM)** | 5 µl |
| **P5 Adapter (20 µM) SEQ ID NO: 1** | 1 µl |
| **P7 Adapter (20 µM) SEQ ID NO: 2** | 1 µl |
| **Genomic DNA (250ng/µl)** | 1.6 µl |
| **H₂O** | 32.4 µl |
| Total | 50 µl Rxn |

2. Adapter extension: Add 0.5 µl GoTaq DNA Polymerase (5U/µl) + 1.5 µl dNTP mix (10 mM).
Incubate at 74°C for 30 min.
Clean (DCC™) with 6:1 volume DNA binding buffer (DBB). Elute in 12 µl elution buffer or water (Zymo Research Corp.).
3. Single-strand extension: The P7 Adapter CGm is a primer that will anneal to the P7 Adapter and extend to create a newly synthesized strand. P7 Adapter CGm contains only methylated cytosines, which allow the newly synthesized strand to be distinguished from the parental strand. This allows only the newly synthesized strand to be amplified during post-bisulfite PCR amplification.
SEQ ID NO: 3:
P7 Adapter: CGm: 5' GTGA /meC/ TGGAGTT /meC/ AGA /meC/ GTGTG /meC/ T /meC/ TT /meC/ /meC/ GATCT-3' 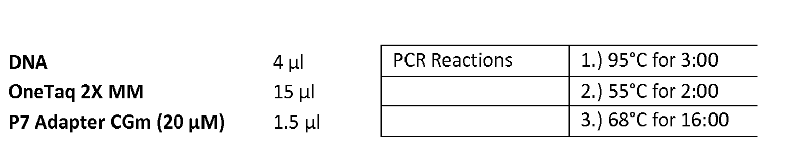 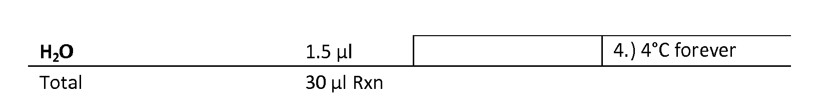
Clean (DCC™) with 6:1 volume DNA binding buffer (DBB). Elute in 15 µl elution buffer (Zymo Research Corp.).
4. Glucosylation of 5hmC reaction: Incubate overnight at 37°C. Clean up with Oligo Clean & Concentrator and elute in 15 µl.

| | **Di-glucosylation** | **+BGT** | **Negative** |
|---|---|---|---|
| **10X GT Rxn Buffer** | 5 µl | 5 µl | 5 µl |
| **10X UDPG** | 5 µl | 5 µl | 5 µl |
| **βGT enzyme (2U/µl)** | 5 µl | 5 µl | - |
| **T6-βGT Enzyme** | 10 µl | - | - |
| **DNA (500 ng total)** | 15 µl | 15 µl | 15 µl |
| **H₂O** | 10 µl | 20 µl | 25 µl |
| **Total** | 50 µl | 50 µl | 50 µl |

5. M.SssI methylation of CpG sites: Incubate at 30°C for 4 h and heat-inactivate at 65°C for 15 min.
After 2 hrs of incubation, add 1 µl of CpG Methylase to drive the reaction.

| | |
|---|---|
| **10X CpG Rxn Buffer** | 2 µl |
| **20X SAM** | 1 µl |
| **DNA** | 13.5 µl |
| **CpG Methylase** | 1 µl |
| **H₂O** | 2.5 µl |
| Total | 20 µl Rxn |

6. Size-select about 150 to about 350 bp fragment on a 2.5% NuSieve/Agarose gel using Zymoclean™ Gel Recovery kit. Elute in 20 µl elution buffer or water (Zymo Research Corp.).
7. Use the EX DNA Methylation-Lightning® Bisulfite kit to bisulfite treat the methylated DNA. Elute the converted DNA in 20µl elution buffer or water (Zymo Research Corp.).
8. Final PCR amplification:

| | | | | |
|---|---|---|---|---|
| **DNA** | 20 µl | | | |
| **P5 Primer (20 µM)** | 0.5 µl | | | |
| **P7 Primer (20 µM)** | 0.5 µl | | | |
| **OneTaq 2X MM** | 25 µl | PCR Reactions | 1.) 94°C for 0:30 | |
| **H₂O** | 4 µl | | 2.) 94°C for 0:30 | \| |
| Total | 50 µl Rxn | | 3.) 58°C for 0:30 | \|X 14 cycles |
| | | | 4.) 68°C for 1:00 | \| |
| | | | 5.) 68°C for 5:00 | |
| | | | 6.) 4°C forever | |

9. Visualize 5 µl aliquot on a
2% Agarose gel and should see a smear at 150bp-350bp.
10. Clean-up amplified library using 6:1 DNA binding buffer (DBB) and elute in 13 µl.

### Example 3: 5hmC Library Preparation Protocol 2

Purpose: Prepare libraries using the strand extension method coupled with enzyme-based modifications for high-throughput sequencing. Use this method to detect 5hmC of tumor versus normal liver samples.
**1.** Digestion and adapter ligation: Incubate at 21°C for 6 hrs and 4°C overnight.

| | |
|---|---|
| **10X NEB Buffer 4** | 5 µl |
| **Mspl (20U/µl**) | 2 µl |
| **T4 Ligase (2000U/µl)** | 1 µl |
| **ATP (lOmM)** | 0.5 µl |
| **P5 Adapter (20 µM) (SEQ ID NO: 1)** | 0.5 µl |
| **P7 Adapter (20 µM) SEQ ID NO: 2)** | 0.5 µl |
| **Genomic DNA (400 total)** | X µl |
| **H₂O** | Y µl |
| Total | 50 µl Rxn |

**2.** Adapter extension: Add 0.5 µl GoTaq DNA Polymerase (5U/µl) + 1.5 µl dNTP mix (10 mM) (Promega Corp.).
Incubate at 74°C for 30 min.
Clean (DCC) with 6:1 volume of DNA binding buffer (DBB). Elute in 12 µl elution buffer or water (Zymo Research Corp.).
**3.** Single-strand extension: The P7 Adapter CGm (SEQ ID NO: 3) is a primer that will anneal to the P7 Adapter and extended to create a newly synthesized strand. The P7 Adapter CGm contains only methylated cytosines, which allow the newly synthesized strand to be distinguished from the parental strand. This allows only the newly synthesized strand to be amplified during post-bisulfite PCR amplification. Clean with DNA Clean and Concentrator (DCC™) with 6:1 volume DNA binding buffer (DBB). Elute in 15 µl elution buffer (DEB). (SEQ ID NO: 3: P7 Adapter, CGm). 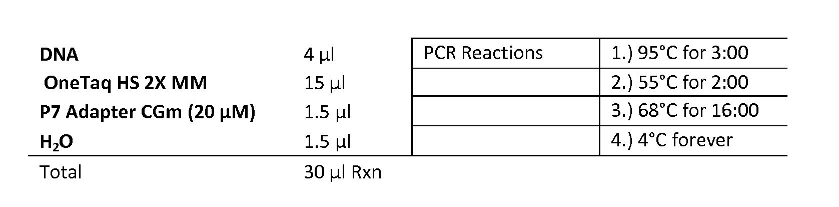
**4.** Glucosylation reaction of 5hmC: Incubate overnight at 37°C. Clean up with Oligo Clean & Concentrator and elute in 15 µl.

| | **Di-glucosylation** | **+BGT** | **Negative** |
|---|---|---|---|
| **10X GT Rxn Buffer** | 5 µl | 5 µl | 5 µl |
| **10X UDPG** | 5 µl | 5 µl | 5 µl |
| **βGT enzyme (2U/µl)** | 5 µl | 5 µl | - |
| **T6-βGT Enzyme** | 10 µl | - | - |
| **DNA (500 ng total)** | 15 µl | 15 µl | 15 µl |
| **H₂O** | 10 µl | 20 µl | 25 µl |
| **Total** | 50 µl | 50 µl | 50 µl |

**5.** M.SssI methylation of CpG sites: Incubate at 30°C for 4 h and heat-inactivate at 65°C for 15 mins.

| | |
|---|---|
| **10X CpG Rxn Buffer** | 2 µl |
| **20X SAM** | 1 µl |
| **DNA** | 13.5 µl |
| **CpG Methylase** | 2 µl |
| **H₂O** | 1.5 µl |
| Total | 20 µl Rxn |

**6.** Size-select about 150 to about 250bp and BOUT 250 to about 350bp fragment on a 2.5% NuSieve/Agarose gel using Zymoclean™ Gel Recovery kit. Elute in 20µl elution buffer (P7 Adapter Hybridization Reaction Volume (µl).
10X Oligo Hybridization Buffer: 5 µl.
Adapter P5 & P7 CGs noA (100 µM): 10 µl.
Adapter P7CG (100µM): 10 µl.
DNase/RNase Free Water: 25 µl.
Total reaction volume : 50 µl.
**7.** Use the EZ DNA Methylation-Lightning® bisulfite kit to bisulfite treat the methylated DNA. Elute the converted DNA in 20 µl elution buffer (Zymo Research Corp.).
**8.** Final PCR amplification:

| | | | | |
|---|---|---|---|---|
| **DNA** | 20 µl | PCR Reactions | 1.) 94°C for 0:30 | |
| **P5 Primer (20 µM) (SEQ ID NO: 1)** | 0.5 µl | | 2.) 94°C for 0:30 | \| |
| | | | 3.) 58°C for 0:30 | \|X 14 cycles |
| **P7 Primer (20 µM) (SEQ ID NO: 2)** | 0.5 µl | | 4.) 68°C for 1:00 | \| |
| | | | 5.) 68°C for 5:00 | |
| **OneTaq (NEB) HS 2X MM** | 25 µl | | 6.) 4°C forever | |
| **H₂O** | 4 µl | | | |
| Total | 50 µl Rxn | | | |

**9.** Visualize 5 µl aliquot on a 2% Agarose gel and should see a smear at 150 bp-350 bp.
**10.** Clean-up amplified library using 6:1 DNA binding buffer (DBB) and elute in 13 µl.

### Example 4: Locus-specific Method: Validating Sequencing Results of Tumor vs. Normal Liver.

Purpose: Develop a locus-specific assay to increase 5hmC sequencing depth and to validate the library sequencing results.
**1.** Single strand extension of specific locus: The 5' end of the extension primer contains a universal sequence (SEQ ID NO: 6: 5'AGATCGGAAGAGCGTCGTGTAGGGAAAGAGTGT-3') that does not align to the human genome and the 3' end is specific to the targeted loci of the human genome. Clean by DNA Clean and Concentrator (DCC™) with 1:2 DNA binding buffer (DBB) and elute in 15 µl elution buffer or water (Zymo Research Corp.). 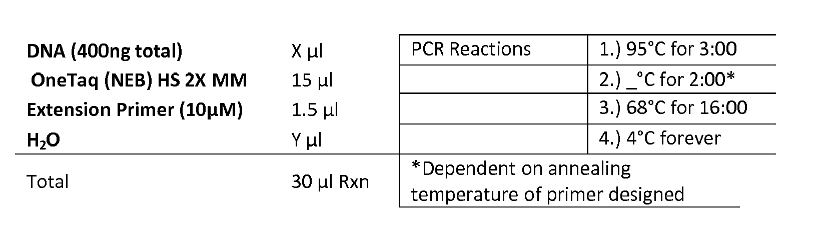
**2.** Glucosylation of 5hmc: Incubate at 37°C overnight. Clean-up with DCC™ and elute in 13 µl elution buffer or water (Zymo Research Corp.).

| | **+BGT** | **Negative** |
|---|---|---|
| **10X GT Rxn Buffer** | 5 µl | 5 µl |
| **10X UDPG** | 5 µl | 5 µl |
| **βGT enzyme (2U/µl)** | 4 µl | - |
| **T6-βGT Enzyme** | - | - |
| **DNA (500 ng total)** | 15 µl | 15 µl |
| **H₂O** | 21 µl | 25 µl |
| **Total** | 50 µl | 50 µl |

**3.** M.SssI methylation of CpG sites: Incubate at 30°C for 4 h and heat-inactivate at 65°C for 15mins.

| | |
|---|---|
| **10X CpG Rxn Buffer** | 2 µl |
| **20X SAM** | 1 µl |
| **DNA** | 13.5 µl |
| **CpG Methylase** | 2 µl |
| **H₂O** | 1.5 µl |
| Total | 20 µl Rxn |

**4.** After the incubation, add 130 µl of EZ DNA Methylation-Lightning™ Conversion Reagent directly to the methylation reaction and follow the kit protocol. Elute in 15 µl elution buffer or water (Zymo Research Corp.).

| | | |
|---|---|---|
| PCR Reactions | 1.) 94°C for 0:30 | |
| | 2.) 94°C for 0:30 | \| |
| | 3.) _°C for 0:30* | \|X 35 cycles |
| | 4.) 68°C for 1:00 | \| |
| | 5.) 68°C for 5:00 | |
| | 6.) 4°C forever | |
| *Dependent on the annealing temperature of the BS Fwd Primer | | |

**5.** PCR amplification:

| | |
|---|---|
| **DNA** | 15 µl |
| **SEQ ID NO: 16 Primer BS Fwd (10 µM)** | 0.8 µl |
| **SEQ ID NO: 17 Primer BS Universal Rev (10 µM)** | 0.8 µl |
| **OneTaq HS 2X MM** | 25 µl |
| **H₂O** | 8.4 µl |
| Total | 50 µl Rxn |

**6.** Load the whole PCR reaction onto an agarose gel and gel excise the fragment for sequencing.

### Example 5: Bioinformatics analysis of samples for significant 5hmC CpG sites for individual CpGs interpreted from sequence daughter strand to parental strand.

The hydroxymethylcytosie (5hmC) calling for each sample was done with the Python pipeline with a filter read out coverage smaller than 5 reads (Zymo Research Corp.). Single 5hmC CpGs for all samples were mapped to a reference genome. Significant individual 5hmC CpG sites were determined by interpretation of sequence reads back to the parental strand. Thus the hydroxymethylation (5hmC) identity and quantitation of the parent DNA strand is calculated from the information from the opposite strand (e.g. A/T for 5hmC versus C/T for C or 5mC). In analysis no β-GT controls allow filitering out of single nucleotide polymorphisms that could add background(e.g. spontaneous A/T not from 5hmC in parental DNA strand). Chromosome regions for humans are found at the UCSC (University of California, Santa Cruz) sequence tracks and CpG island tracks (http://genome.ucsc.edu/cgi-bin/hgTables?hgsid=328616477) (Kent W.J., et al., 2002) with the Feb. 2009 human reference sequence GRCh 37/hgl9 assembly reference. For each chromosomal region analysis for the whole genome the methylation ratio of all detected CpG sites of each sample are obtained. If there is no CpG site for a region it is missing data. If a chromosomal region is missing data it is discarded if more than 20% of the data is missing for a sample. CpGs in each chromosomal region and were deemed significant if the p value was <0.05. 5hmC CpGs in each chromosomal region with higher p-values, < 0.05, were discarded in this analysis. Feb. 2009 human reference sequence GRCh 37/hg19 assembly incorporated herein by reference in its entirety. Hierarchical Clustering analysis can be done for samples and can be shown graphically via heat maps (data not shown) performed with the Python suite of programs (Zymo Research Corp.). Distinct 5hmC patterns can be determined by such analysis.

**Example 6: Whole Genome Sequencing.** The following protocol is suitable for analysis of 5hmC in an entire genome of a desired species (e.g. humans).

### I. Adapter Hybridization: Preparation of adapters used for library preparation.

**1.** Prepare the P5 and P7 Adapters at 20µM through hybridization of the short and long oligos.

| **P5 Adapter Hybridization Reaction** | **Volume (µl)** |
|---|---|
| 10X Oligo Hybridization Buffer | 5 µl |
| Adapter P5 & P7 CGs no A (100 µM) | 10 µl |
| SEQ ID NO: 7 | |
| SEQ ID NO: 9 | |
| Adapter P5CG (100µM) | 10 µl |
| SEQ ID NO: 7 | |
| DNase/RNase Free Water | 25 µl |
| **Total Volume** | **50 µl** |

| **P7 Adapter Hybridization Reaction** | **Volume (µl)** |
|---|---|
| 10X Oligo Hybridization Buffer | 5 µl |
| Adapter P5 & P7 CGs_no A (100 µM) | 10 µl |
| SEQ ID NO: 7 | |
| SEQ ID NO: 9 | |
| Adapter P7CG (100µM) | 10 µl |
| SEQ ID NO: 3 | |
| DNase/RNase Free Water | 25 µl |
| **Total Volume** | **50 µl** |

**2.** Run the reaction in the thermal cycler with the following conditions:
95°C for 10 sec.
Ramp to 12°C at 0.1°C/sec.
12°C for 1 min.
4°C (indefinitely).
**3.** Store the adapters at -20°C.

### II. Whole-genome library preparation protocol.

1. DNA Fragmentation
   Dilute dsDNA Sherase Plus to 0.25U/ µl: 5 µl of Shearse (1U/µl) + 15 µl H2O (Zymo Research Corp.). This will generate fragments from about 100bp - 500bp, which is a preferred size for library construction and sequencing.
   Genomic DNA (500 ng): 0.1-1 µl
   5X Sherase Rxn Buffer: 4.0 µl
   H₂O:13.9-13 µl
   Atlantis sherase (0.25U/µl): 2 µl
   20 µl total reaction volume.
   Incubate at 42°C for 20 min and heat-inactivate at 65°C for 5 min.
   Clean reaction with DNA Clean and Concentrator™ (DCC™: Zymo Research Corp.) 1:5 with DNA Binding Buffer (DBB) and elute in 2 X 13 µl of elution buffer or water.
2. End-blunting
   10X (NEB Buffer 4: 3 µl
   dNTPs (2.5 mM each): 0.75 µl
   NEB Klenow fragment (3'->5' exo-) (5U/ µl): 0.5 µl
   NEB T4 DNA Polymerase (3U/ µl): 0.5 µl
   Fragmented DNA: 25.25 µl
   30 µl total reaction volume.
   Incubate at room temperature (RT) for 15 min.
   Clean reaction with DCC™ 1:5 with DBB and elute in 2 X 12 µl of elution buffer or water.
**3.** A-tailing.
   End-blunt DNA: 24 µl.
   A-tailing mix (1.25 mM dATP, 0.125 mM dNTP): 1.5 µl
   10X NEBuffer 4: 3 µl
   NEB Klenow fragment (3'->5' exo-) (5U/ µl): 1.5 µl.
   30 µl total reaction volume.
   Incubate at 37° for 1 hr.
   Clean reaction with DCC™ 1:5 with DBB and elute in 2 X 7.5 µl of elution buffer or water.
**4.** Adapter Ligation.
   A-tailed DNA fragments: 15 µl.
   NEB T4 10X Ligase Buffer: 2 µl.
   NEB T4 Ligase (400 U/ µl): 1 µl.
   P5 (SEQ ID NO: 1) Adapter (6 µM): 1 µl.
   P7 P5 (SEQ ID NO: 2) Adapter (6 µM): 1 µl.
   20 µl total reaction volume.
   Incubate at 16°C for 12 hrs.
5. Adapter Extension
   Add 0.5 µl GoTaq Polymerase (5 U/µl) and 1.5 µl dNTPs mix (10 mM). Incubate at 74°C for 30 min.
   Clean reaction with DCC™ 1:5 with DBB and elute in 13.5 µl of elution buffer or water.
6. Single-strand extension with methylated P7 Adapter: synthesize the new strand to mirror the parental strand.
   Adapterized DNA: 13.5 µl.
   OneTaq 2X HS MM: 15 µl.
   P7 Adapter CGm (SEQ ID NO: 3) (20 µM): 1.5 µl.
   30 µl total reaction volume.
   Incubate at:
   1. 94°C for 3 min.
   2. 55°C for 2 min.
   3. 68°C for 16 min.
   4. 4°C (indefinitely).
   Clean reaction with DCC™ 1:5 with DBB and elute in 15 µl of elution buffer or water.
7. Glucosylation of 5hmC.
   Include α-BGT control: omit 10X UDPG and β-GT enzymes from reaction.
   DNA: 15 µl
   10X NEBuffer 4: 5 µl
   10X UDPG: 5 µl
   β-GT enzyme: 4 µl
   H₂O: 21 µl
   50 µl total reaction volume.
   Incubate at 37°C for 12 hrs. Clean-up with Oligo Clean and Concentrator™ (DCC: Zymo Research Corp.). Elute in 2 X 13.5 µl of elution buffer or water.
8. Optional Purification of Glucosylated DNA with Proteinase K treatment.
   Add 50 µl 2X Digestion Buffer and 1 µl of Proteinase K (∼20mg/ml) directly to the reaction and incubate at 55°C for 1 hr (do not let the reaction go to 4°C because the digestion buffer will begin to precipitate).
   Clean-up with 500µl of ChIP Binding Buffer (Zymo Research Corp.) and wash the column twice with 200 µl DNA Wash Buffer. Elute in 13.5 µl ZEB and 13.5 µl H₂O.
9. M.SssI Methylation: methylate CpG sites not mirroring a glucosyl-5hmC site
   Split the library in two and prepare two reactions to ensure complete methylation.
   10X CpG Rxn Buffer: 2 µl
   20X SAM: 1 µl
   CpG Methylase, M.SssI (Zymo Research Corp.) (4U/µl): 2 µl
   DNA Library: 13.5 µl
   H₂O: 1.5 µl
   20 µl total reaction volume.
   Incubate at 30°C for 4 hrs and heat inactivate at 65°C for 15 min.
**10.** Bisulfite Conversion
   Follow EZ DNA Methylation-Lightning™ Bisulfite Conversion Kit protocol (Zymo Research Corp.): Add 130 µl of conversion reagent directly to the M.SssI reaction. During the binding step, pool the two reactions together by doing two binding.
   Elute in 20 µl elution buffer or water.
**11.** Library PCR Amplification: Selectively amplify the newly synthesized strand
   BS Converted DNA Library: 20 µl
   OneTaq (New England BioLabs Inc.) 2X HS MM: 25 µl
   P5 Primer (SEQ ID NO: 1) (20 µM): 0.5 µl
   P7 Primer (SEQ ID NO: 2) (20 µM): 0.5 µl
   H₂O: 4 µl
   50 µl total reaction volume.
   PCR Conditions:
   1. 94°C for 30 sec
   2. 94°C for 30 sec
   3. 58°C for 30 sec
   4. 68°C for 3 min
   5. 68°C for 5 min
   6. 4°C forever
   Perform the above PCR regime for 15 cycles.
**12.** Library Quality Control: Run a 5 µl aliquot of the PCR reaction on a 2% agarose gel to ensure the library has amplified (the expected smear size should be between about 100 to about 500bp).
**13.** Purification of completed libraries: Purify using the DNA Clean & Concentrator with 1:5 DBB and elute in 15 µl.
**14.** Perform analysis of library via Next-Gen sequencing (Illumina) or other available platform as described above.
**15.** Bioinformatics analysis to identify and quantitate the entire genome content of 5hmC is performed as described above.

### Example 7: Titration of Genomic DNA for Genome-Wide Library Preparation.

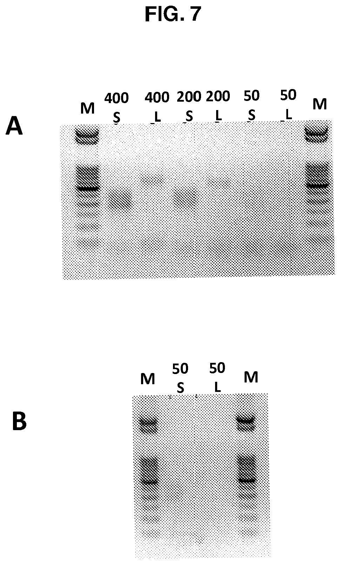

Determine the amount of genomic DNA required for genome-wide library preparation. Representative libraries were prepared as described above from various genomic DNA input amounts of: 400 ng, 200 ng, and 50 ng. Results show libraries can be generated from as low as 50ng; the band for the 50ng input is much fainter than the 400ng and 200ng input but with an additional 2 cycles of amplification, the intensity of the bands can be increased (See FIG. 7A and 7B). Note "S" indicates short fragments that were gel selected from about 150bp to about 250bp and "L" indicates long fragments that were gel selected from about 250bp to about 350bp.

### Example 8: Spike-in Controls for Genome-Wide Library Preparation.

Synthesize spike-in controls was performed to determine the efficiency of the enzymatic reactions (i.e. glucosylation and methylation) during library preparation.

Spike-in controls were synthesized from *E. coli* DNA by designing a forward and reverse primer that flanked CCGG sites but allowed the 3' end of the primer to overlap the CCGG site. Two forward primers were synthesized for a single control; one with a 5hmC at the internal cytosine of the CCGG site (i.e. SEQ ID NO: 18, C/5hmC/GG) and one without any modification (SEQ ID NOs: 19-29). After amplification of the controls, the amplicons with the single 5hmC site and those without any modifications were pooled with varying levels of 5hmC. Four different spike-in controls with varying levels of 5hmC (i.e. 0%, 5%, 50%, and 100%) were generated and spiked into genomic DNA. Genome-wide libraries were then prepared and sequenced. Sequencing results shows that the calculated 5hmC level from Mirror-Bisulfite Sequencing Analysis (Mirror™ Bisulfite Analysis, or Mirror-BS) corresponds with the theoretical value of 5hmC in the spiked DNA indicating quantitation is reliable over the range tested.

### REFERENCES

WO/2010/114821
U.S. Patent: 5,786,146
U.S. Patent Publication: 2010/0304992
U.S. Patent Publication: 2011/0046009
U.S. Patent Publication: 2009/0305241
U.S. Patent Publication: 2012/0149023
PCT Pub. No.: WO/2010/114821
U.S. Patent Application: 12,963,272
U.S. Patent Application: 13/392.286
U.S. Patent Application: 13/933,051
Booth M. J. et al., Science 336 (6083): 934-7, 2011.
Branco M.R. et al., Nat. Rev. Genetics 13: 7-13, 2012.
Callinan, Hum. Mol. Genet., 15:R95-101, 2006.
Fraga, BioTechniques, 33(3):632, 634, 636-49, 2002.
Globisch D et al. (December 2010), PLoS ONE 5 (12): e15367.
Gyarmati, P. et al, Journal of Biotechnology, Volume 168, Issue 1, Pages 95-100, 2013.
He, Y. F. et al. Sep 2; 333, 1303-1307, 2011.
Ito S. et al., Science Sep. 2; 333,1300-1303, 2011.
Jin S., et al., Cancer Res 71 (24): 7360-7365, 2011.
Jones P.S., et al., Nat. Genet., 21(2):163-7, 1999.
Kent W.J., et al., Genome Research, 12: 996-1106, 2002.
Kinney, S. M., et al. The Journal of Biological Chemistry, Vol. 286, No. 28, pp-24685-24693, 2011.
Kornberg S. R. et al. The Journal of Biological Chemistry, Vol. 236, No. 5, 1961.
Laird, Nat. Rev. Cancer, 3(4):253-66, 2003.
Longo, M. C., et al., Gene 93 (1): 125-128, 1990.
Ping, Xiao-Li et al. Cell Research, 24:177-189, 2014
Robertson, A. B. et al., Nature Protocols, Vol. 7, No. 2, 2012.
Sambrook et al., In: Molecular Cloning-A Laboratory Manual, 1989.
Xiao-Li Ping et al. 2014) and 5-formylcytosine (5fC) and 5-carboxylcytosine (5caC) (Ito S. et. al. 2011; Yu-Fei He et. al. 2011
The Genome Sequencing Consortium. Initial sequencing and analysis of the human genome. Nature. 2001 Feb 15;409(6822):860-921.

## Claims

1. A method for identifying hydroxymethylcytosine residues in a DNA sample comprising:
synthesizing a DNA strand complementary to each parental DNA strand of a DNA sample with a methylated primer;
contacting the DNA sample with a glucosyltransferase, wherein hydroxymethylcytosine residues have at least one glucose added generating glucosyl-hydroxymethylcytosine residues;
contacting the DNA sample with at least one methyltransferase, wherein unmodified cytosine residues have a methyl group added, except for cytosine residues adjacent to glucosyl-hydroxymethylcytosine residues and on the opposite parental DNA strand which remain unmethylated;
contacting the DNA sample with bisulfite, wherein unmodified cytosine residues are converted to uracil;
preferentially amplifying the newly synthesized strands from the DNA sample with a forward and a methylated reverse primer generating amplification products;
analyzing each of the amplification products to determine its sequence; and
identifying the hydroxymethylcytosine residues in each of the amplification products through interpreting adjacent non-converted cytosine residues on the opposite DNA strand.

2. The method of claim 1, wherein the methylated primer is a P7 primer or CS1 primer.

3. The method of claim 1, wherein the DNA sample further comprises a plurality of genomic DNA fragments.

4. The method of claim 1, wherein the genomic DNA fragments each contain at least one adapter on each end.

5. The method of claim 4, wherein the adapters are commercially available and are chosen from the group consisting of P5, P7, CS1, and CS2.

6. The method of claim 1, wherein the forward and reverse primers are selected from the group consisting of a P7 primer, a P5 primer, a CS1/PE1 primer, and a CS2/PE2 primer.

7. The method of claim 1, wherein the step of preferentially amplifying the newly synthesized strands from the DNA sample is performed with at least one set of locus specific primers generating at least one locus specific amplicon.

8. The method of claim 7, wherein the step of synthesizing a DNA strand complementary to each parental DNA strand of a DNA sample further comprises performing the synthesis with a methylated primer with a universal sequence of at least about 20 bp to about 32 bp at the 5 prime end.

9. The method of claim 1, wherein the step of identifying the hydroxymethylcytosine residues in each of the amplification products further comprises mapping sequence reads to a reference genome with a computer with software wherein sequence information from the newly synthesized strand identifies the hydroxymethylcytosine residues on the parent strand.

10. The method of claim 1, further comprising quantitating the amount of hydroxymethylcytosine residues in the DNA sample.

## Patentansprüche

1. Verfahren zur Identifizierung von Hydroxymethylcytosinresten in einer DNA Probe, aufweisend:
Synthetisieren eines DNA Stranges, der komplementär ist zu jedem Eltern-DNA Strang einer DNA Probe, mit einem methylierten Primer;
Kontaktieren der DNA Probe mit einer Glycosyltransferase, wobei den Hydroxymethylcytosinresten mindestens eine Glucose derart zugefügt wird, dass Glucosyl-Hydroxymethylcytosinreste generiert werden;
Kontaktieren der DNA Probe mit mindestens einer Methyltransferase, wobei nicht-modifizierten Cytosinresten mindestens eine Methylgruppe zugefügt wird, mit Ausnahme von Cytosinresten, die sich neben Glucosyl-Hydroxymethylcytosinresten und auf dem gegenüberliegenden Eltern-DNA Strang befinden, welche unmethyliert bleiben;
Kontaktieren der DNA Probe mit Bisulfit, wobei nicht-modifizierte Cytosinreste zu Urazil konvertiert werden;
bevorzugt amplifizieren der neu synthetisierten Stränge von der DNA Probe mit einem Vorwärts- und einem methylierten Rückwärts-Primer, wobei Amplifikationsprodukte generiert werden;
Analysieren der Amplifikationsprodukte zur Bestimmung deren Sequenz; und
Identifizieren der Hydroxymethylcytosinreste in jedem der Amplifikationsprodukte mittels Interpretation der benachbarten nicht-konvertierten Cytosinreste auf dem gegenüberliegenden DNA Strang.

2. Verfahren nach Anspruch 1, wobei der methylierte Primer ein P7-Primer oder ein CS1-Primer ist.

3. Verfahren nach Anspruch 1, wobei die DNA Probe weiterhin eine Mehrzahl von genomischen DNA-Fragmenten umfasst.

4. Verfahren nach Anspruch 1, wobei die genomischen DNA-Fragmente jeweils mindestens einen Adapter an jedem Ende enthalten.

5. Verfahren nach Anspruch 4, wobei die Adapter kommerziell erhältlich sind und ausgewählt sind aus der Gruppe bestehend aus P5, P7, CS1 und CS2.

6. Verfahren nach Anspruch 1, wobei die Vorwärts- und Rückwärts-Primer ausgewählt sind aus der Gruppe bestehend aus einem P7-Primer, einem P5-Primer, einem CS1/PE1-Primer und einem CS2/PE2-Primer.

7. Verfahren nach Anspruch 1, wobei der Schritt des bevorzugten amplifizieren der neu synthetisierten Stränge der DNA Probe mit mindestens einem Set locus-spezifischer Primer durchgeführt wird, wobei mindestens ein locus-spezifisches Amplikon generiert wird.

8. Verfahren nach Anspruch 7, wobei der Schritt des synthetisierens eines DNA Stranges komplementär zu jedem Eltern-DNA-Strang einer DNA Probe weiterhin umfasst die Durchführung der Synthese mit einem methylierten Primer mit einer universellen Sequenz von mindestens ungefähr 20 bp bis ungefähr 32 bp am 5-Ende.

9. Verfahren nach Anspruch 1, wobei der Schritt der Identifizierung der Hydroxymethylcytosinreste in den Amplifikationsprodukten weiterhin aufweist, dass Kartieren von gelesenen Sequenzen mit einem Referenzgenom mittels eines Computers mit Software, wobei Sequenzinformation des neu synthetisierten Stranges die Hydroxymethylcytosinreste des Eltern-Stranges identifizieren.

10. Verfahren nach Anspruch 1, weiterhin aufweisend die Quantifizierung der Menge der Hydroxymethylcytosinreste in der DNA Probe.

## Revendications

1. Procédé d'identification de résidus hydroxyméthylcytosine dans un échantillon d'ADN comprenant :
la synthèse d'un brin d'ADN complémentaire de chaque brin d'ADN parental d'un échantillon d'ADN avec une amorce méthylée ;
la mise en contact de l'échantillon d'ADN avec une glucosyltransférase, dans lequel les résidus hydroxyméthylcytosine ont au moins un glucose ajouté de façon à générer des résidus glucosyl-hydroxyméthylcytosine ;
la mise en contact de l'échantillon d'ADN avec au moins une méthyltransférase, dans lequel les résidus cytosine non modifiés ont un groupe méthyle ajouté, sauf pour les résidus cytosine adjacents aux résidus glucosyl-hydroxyméthylcytosine et sur le brin d'ADN parental opposé qui restent non méthylés ;
la mise en contact de l'échantillon d'ADN avec du bisulfite, dans lequel des résidus cytosine non modifiés sont convertis en uracile ;
l'amplification préférentielle des brins nouvellement synthétisés à partir de l'échantillon d'ADN avec une amorce sens et une amorce antisens méthylée de façon à générer des produits d'amplification ;
l'analyse de chacun des produits d'amplification pour déterminer sa séquence ; et
l'identification des résidus hydroxyméthylcytosine dans chacun des produits d'amplification par interprétation de résidus cytosine non convertis adjacents sur le brin d'ADN opposé.

2. Procédé selon la revendication 1, dans lequel l'amorce méthylée est une amorce P7 ou une amorce CS1.

3. Procédé selon la revendication 1, dans lequel l'échantillon d'ADN comprend en outre une pluralité de fragments d'ADN génomique.

4. Procédé selon la revendication 1, dans lequel les fragments d'ADN génomique contiennent chacun au moins un adaptateur sur chaque extrémité.

5. Procédé selon la revendication 4, dans lequel les adaptateurs sont commercialisés et sont choisis dans le groupe constitué de P5, P7, CS1 et CS2.

6. Procédé selon la revendication 1, dans lequel les amorces sens et antisens sont choisies dans le groupe constitué d'une amorce P7, une amorce P5, une amorce CS1/PE1 et une amorce CS2/PE2.

7. Procédé selon la revendication 1, dans lequel l'étape d'amplification préférentielle des brins nouvellement synthétisés à partir de l'échantillon d'ADN est conduite avec au moins un ensemble d'amorces spécifiques à un locus de façon à générer au moins un amplicon spécifique à un locus.

8. Procédé selon la revendication 7, dans lequel l'étape de synthèse d'un brin d'ADN complémentaire de chaque brin d'ADN parental d'un échantillon d'ADN comprend en outre la conduite de la synthèse avec une amorce méthylée avec une séquence universelle d'au moins environ 20 pb à environ 32 pb à l'extrémité 5 prime.

9. Procédé selon la revendication 1, dans lequel l'étape d'identification des résidus hydroxyméthylcytosine dans chacun des produits d'amplification comprend en outre la mise en correspondance de lectures de séquence avec un génome de référence à l'aide d'un ordinateur avec un logiciel dans lequel des informations de séquence du brin nouvellement synthétisé identifient les résidus hydroxyméthylcytosine sur le brin parent.

10. Procédé selon la revendication 1, comprenant en outre la quantification de la quantité de résidus hydroxyméthylcytosine dans l'échantillon d'ADN.
